# EUROPEAN PATENT APPLICATION

(11) **EP 4 517 329 A1**
(43) Date of publication of application: **05.03.2025**
(21) Application number: 23796128.9
(22) Date of filing: 12.04.2023
(51) Int. Cl.: G01N 35/02

(54) **TESTING DEVICE**

(30) Priority: 28.04.2022 JP 2022075197
(71) Applicant: FUJIFILM Corporation, Tokyo 106-8620 (JP)
(72) Inventor: SUGAYA, Takuo, Ashigarakami-gun, Kanagawa 258-8538 (JP)
(74) Representative: Dehns Germany Partnerschaft mbB
(86) International application number: PCT/JP2023/014932
(87) International publication number: WO 2023/210384

(57) **Abstract**

Provided is an examination apparatus including a detection unit configured to execute a detecting process for detecting an examination target substance in a specimen, a suction-discharge mechanism configured to execute a mixing process for mixing particles and a liquid by repeating suction and discharge using a nozzle inserted in a reaction cell, the mixing process being a process which is performed before the detecting process and performed on the specimen to be subjected to the detecting process, and a processor configured to acquire information on a color of a mixed liquid in the nozzle, which contains the particles and the liquid mixed in the reaction cell and is suctioned from the reaction cell to the nozzle, and determine a mixing state of the mixed liquid in the reaction cell from the information on the color.

## Description

### BACKGROUND OF THE INVENTION

### 1. Field of the Invention

The present disclosure relates to an examination apparatus.

### 2. Description of the Related Art

An examination apparatus which quantitatively or qualitatively detects a target substance in a specimen has been known. Such an examination apparatus mainly utilizes an immunoassay principle, and examples thereof include a chemiluminescent enzyme immunological analysis apparatus and a fluorescence immunological analysis apparatus (for example, JP2016-85093A).

In such an examination apparatus, a detecting process of detecting a target substance in a specimen is performed by detecting luminescence or fluorescence based on a label such as an enzyme label and a fluorescent label, which has been imparted to the target substance in the specimen by an immunoreaction. In addition, before such a detecting process of the target substance, a pre-process such as the labeling the target substance in the specimen is performed on the specimen. In some cases, the examination apparatus is configured to automatically execute the pre-process and the detecting process and output a detection result in a case where the pre-process and the detecting process are automated and a specimen collection container accommodating the collected specimen is loaded.

In order to impart a label to the target substance in the specimen, for example, such an automated examination apparatus performs the following process using magnetic particles. First, in a reaction cell, magnetic particles modified with a first binding substance (for example, a primary antibody) for specifically binding to the target substance (for example, an antigen) are mixed with the specimen, so that the target substance and the first binding substance are bound to each other to generate an immune complex. As a result, the target substance is captured by the magnetic particles through the first binding substance. Thereafter, the generated immune complex is separated from the immune complex and a component derived from the specimen (unreacted substance) which does not form the immune complex, that is, so-called Bound/Free (B/F) separation is performed. In a case of the B/F separation, the mixed liquid is suctioned in a state in which the magnetic particles are temporarily adsorbed to an inner wall surface of the reaction cell by a magnet disposed outside the reaction cell.

Thereafter, a cleaning liquid is discharged to the reaction cell, and the mixed liquid is suctioned and discharged in a state in which the cleaning liquid and the magnetic particles are mixed, whereby the magnetic particles are cleaned. Next, a labeling reagent containing a second binding substance (for example, a secondary antibody) for specifically binding to the target substance, which has been bound to the label, is mixed with the magnetic particles. As a result, the target substance captured on the magnetic particles through the first binding substance and the second binding substance are bound to each other, so that a sandwich type immune complex in which the target substance is interposed between the first binding substance and the second binding substance is generated. Thereafter, the magnetic particles are cleaned again by mixing the cleaning liquid and the magnetic particles for the B/F separation. In a case where the label is an enzyme label, the magnetic particles and a reagent containing a luminescent substrate are further mixed and used for the examination process.

In such a manner, in the automated examination apparatus, a mixing process of mixing particles and a liquid, such as mixing of magnetic particles and a specimen, mixing of magnetic particles and a cleaning liquid, mixing of magnetic particles and a labeling reagent, and mixing of magnetic particles and a luminescent reagent, is executed.

In the mixing process, first, a sample solution is suctioned from a specimen collection container by a built-in sampling nozzle, and the suctioned sample solution is discharged to the reaction cell loaded in the examination apparatus. Then, in the reaction cell, the particles and the liquid are mixed with each other as described above.

### SUMMARY OF THE INVENTION

As described above, in a case where the mixing process including the step of mixing the particles and the liquid is performed with the examination procedure in the examination apparatus, the detecting process may be performed in a state in which the mixing of the particles and the liquid has not been sufficiently achieved. In a case where the mixing of the particles and the liquid is insufficient, there is a risk that an examination result is affected, and reliability of quantitativeness of the examination result is reduced. Therefore, it is desired to suppress failure in mixing of the particles and the liquid, but the mixing process of mixing the particles and the liquid is executed in the examination apparatus, so that it is difficult to grasp a mixing state of the particles and the liquid.

An object of the present disclosure is to provide an examination apparatus capable of grasping a mixing state of particles and a liquid.

An examination apparatus according to the present disclosure includes a detection unit for executing a detecting process for detecting an examination target substance in a specimen; a suction-discharge mechanism for executing a mixing process for mixing particles and a liquid by repeating suction and discharge using a nozzle inserted in a reaction cell, the mixing process being a process which is performed before the detecting process and performed on the specimen to be subjected to the detecting process; and a processor for acquiring information on a color of a mixed liquid in the nozzle, which contains the particles and the liquid mixed in the reaction cell and is suctioned from the reaction cell to the nozzle, and determine a mixing state of the mixed liquid in the reaction cell from the information on the color.

The processor may be configured to derive a density of the color from the information on the color of the mixed liquid and configured to that the mixing state is defective in a case where the density of the color is out of a preset regulation range.

A nozzle imaging camera configured to optically image the nozzle may be provided, in which the processor is configured to acquire an image of the nozzle containing the mixed liquid imaged by the nozzle imaging camera, the image including the information on the color of the mixed liquid.

In a case where the mixing state is determined to be defective, the processor may be configured to acquire information on a color of the mixed liquid in the reaction cell, and configured to re-determine the mixing state of the mixed liquid based on the information on the color of the mixed liquid in the reaction cell.

A reaction cell imaging camera configured to optically image the reaction cell may be provided, in which, in a case where the mixing state is determined to be defective, the processor may be configured to image the mixed liquid in the reaction cell by controlling the reaction cell imaging camera, and acquire an image of the mixed liquid in the reaction cell, including the information on the color of the mixed liquid in the reaction cell. **In** addition, the processor may be configured to re-determine the mixing state based on shading in the image of the mixed liquid in the reaction cell, the shading being generated due to dispersion of the particles in the mixed liquid.

The processor may be configured to execute the mixing process by controlling the suction-discharge mechanism to repeat the suction and discharge in the reaction cell a preset number of times using the nozzle, configured to image, by controlling the nozzle imaging camera, the nozzle in a state in which the mixed liquid is suctioned in last among the preset number of times or the nozzle after the mixed liquid is discharged last, and configured to acquire an image of the nozzle imaged by the nozzle imaging camera.

The processor may be configured to image the nozzle after the last discharge by controlling the nozzle imaging camera, configured to acquire an image of the nozzle after the last discharge, configured to, in a case where the mixing state of the mixed liquid is determined to be favorable, derive a liquid amount of a mixed liquid remaining in the nozzle after the last discharge from the image of the nozzle after the last discharge, and configured to correct a detection result of the examination target substance based on the liquid amount of the mixed liquid and a previously acquired calibration residual amount of the liquid, which is an amount remaining in the nozzle after discharge for calibration by performing suction and discharge of the liquid.

A notification unit configured to issue a notification of an error may be provided, in which, in a case where the mixing state is determined to be defective, the processor may be configured to notify an error by the notification unit and terminate the examination of the specimen.

The nozzle may be a sampling nozzle which suctions the specimen and discharges the specimen into the reaction cell, and the processor may be configured to acquire, as a first image, an image of the nozzle containing the specimen, after the specimen is suctioned by the nozzle and before the specimen is discharged into the reaction cell, and acquire, as a second image, an image of the nozzle after the mixing process is executed by repeating the suction and discharge a preset number of times in the reaction cell by the nozzle and the mixed liquid is discharged in last among the preset number of times, and configured to correct a detection result based on the first image and the second image in a case where the mixing state of the mixed liquid is determined to be favorable.

The processor may be configured to acquire an image of the nozzle during the mixing process, and configured to derive a mixing state of the mixed liquid during the mixing process based on the image of the nozzle during the mixing process.

The processor may be configured to increase, by controlling the suction-discharge mechanism, the preset number of times of the suction and discharge in a case where the derived mixing state of the mixed liquid during the mixing process is determined not to satisfy a preset state.

The processor may be configured to image a moving image of the nozzle during the mixing process by the nozzle imaging camera.

The nozzle imaging camera may be capable of simultaneously imaging the reaction cell.

The examination target substance in the specimen may be detected by an antigen-antibody reaction.

The particles may be magnetic particles modified with a binding substance specifically bound to the examination target substance.

According to the technology of the present disclosure, it is possible to grasp a state of a mixture of particles and a liquid.

### BRIEF DESCRIPTION OF THE DRAWINGS

Fig. 1 is a schematic diagram showing an overall configuration of an examination apparatus according to an embodiment.
Fig. 2A is a top view of a cartridge, and Fig. 2B is a front view thereof.
Fig. 3 is a diagram showing an examination procedure.
Fig. 4 is a schematic diagram showing a disposition of a nozzle imaging camera and a reaction cell imaging camera.
Fig. 5 is a schematic diagram showing images of a nozzle containing a mixed liquid.
Fig. 6 is a schematic diagram showing images in which colors of the mixed liquid contained in the nozzle are changed.
Fig. 7 is a diagram showing a first examination flow in the examination apparatus.
Fig. 8 is a diagram showing a second examination flow in the examination apparatus.
Fig. 9 is a diagram showing a third examination flow in the examination apparatus.

### DESCRIPTION OF THE PREFERRED EMBODIMENTS

Hereinafter, an examination apparatus 10 according to the embodiment of the present disclosure will be described with reference to the drawings. Constituent elements indicated by the same reference numeral in the drawings mean the same constituent element. However, unless otherwise specified in the specification, each constituent element is not limited to one and may be plural.

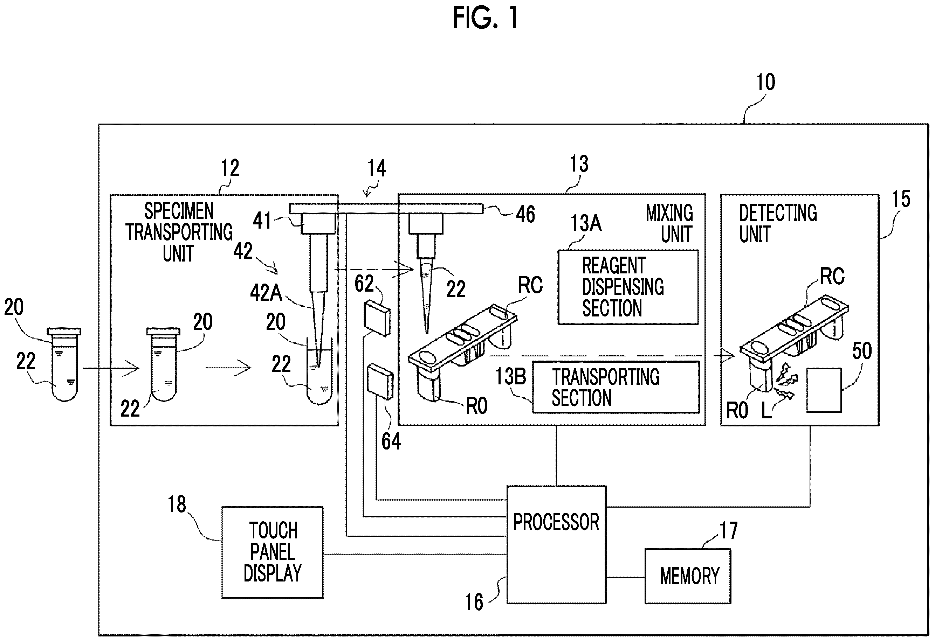

Fig. 1 is a schematic diagram showing an overall configuration of the examination apparatus 10 according to the embodiment of the present disclosure. As an example, the examination apparatus 10 is an immunological analysis apparatus which detects an examination target substance in a specimen 22 collected from a biological body by an antigen-antibody reaction. The examination apparatus 10 optically detects the examination target substance in the specimen 22 using a cartridge RC having a reaction cell R0, and outputs an examination result. As an example, the examination apparatus 10 according to the present example performs an examination based on a chemiluminescent enzyme immunoassay method.

As an example, the specimen 22 is a body fluid such as blood, collected from the biological body. In a case where the specimen 22 is blood, the specimen 22 may be whole blood, blood plasma, serum, or the like. In addition, a centrifuge may be provided in the examination apparatus 10 to extract blood plasma or serum from whole blood. Furthermore, the examination target substance A which can be contained in the specimen 22 is an antigen, an antibody, a protein, a low-molecular-weight compound, or the like. The specimen 22 is not limited to the blood, and may be a substance collected from the biological body, such as urine and body fluid. A specimen collection container 20 which accommodates the specimen 22 to be examined is loaded into the examination apparatus 10 and is subjected to examination.

The cartridge RC is attachably and detachably loaded into the examination apparatus 10. The cartridge RC is a single-use type used once for one specimen 22. As an example, the cartridge RC includes all reagent necessary for the examination of the specimen 22. In addition, a plurality of sets of the specimen collection container 20 and the cartridge RC may be collectively loaded into the examination apparatus 10 such that the examination apparatus 10 can continuously process examinations for the plurality of the specimens 22.

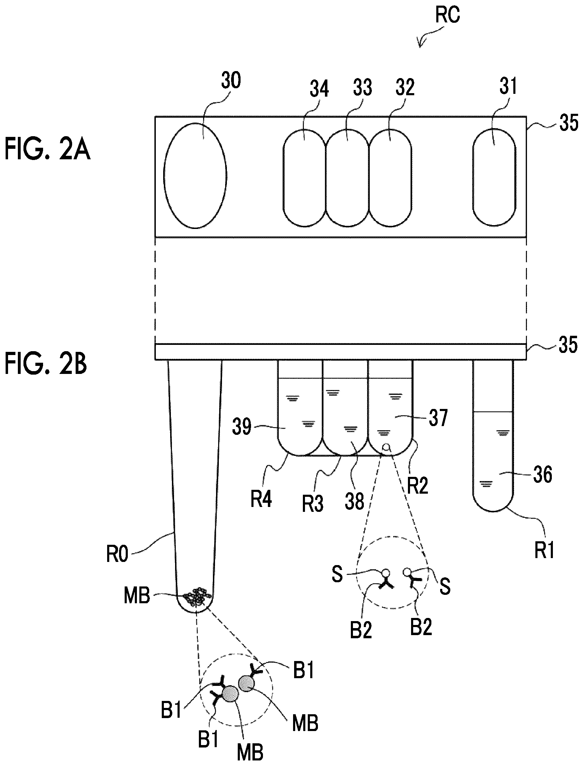

Figs. 2A and 2B are schematic views of an example of the cartridge RC, in which Fig. 2A is a top view of the cartridge RC and Fig. 2B is a front view of the cartridge RC. The cartridge RC includes a plate-shaped connection portion 35 having five openings 30 to 34, and five tubular cells R0 to R4 each having one end of each of the openings 30 to 34 and extending downward to include the reaction cell R0. The cartridge RC has a configuration in which the plurality of cells R0 to R4 are integrated by the connection portion 35. Among the plurality of cells R0 to R4, the reaction cell R0 and the cell R1 disposed at both ends are longer than the other cells R2 to R4. The reaction cell R0 is the longest. The openings 30 to 34 of the cartridge RC are covered with a sealing film (not shown) before use.

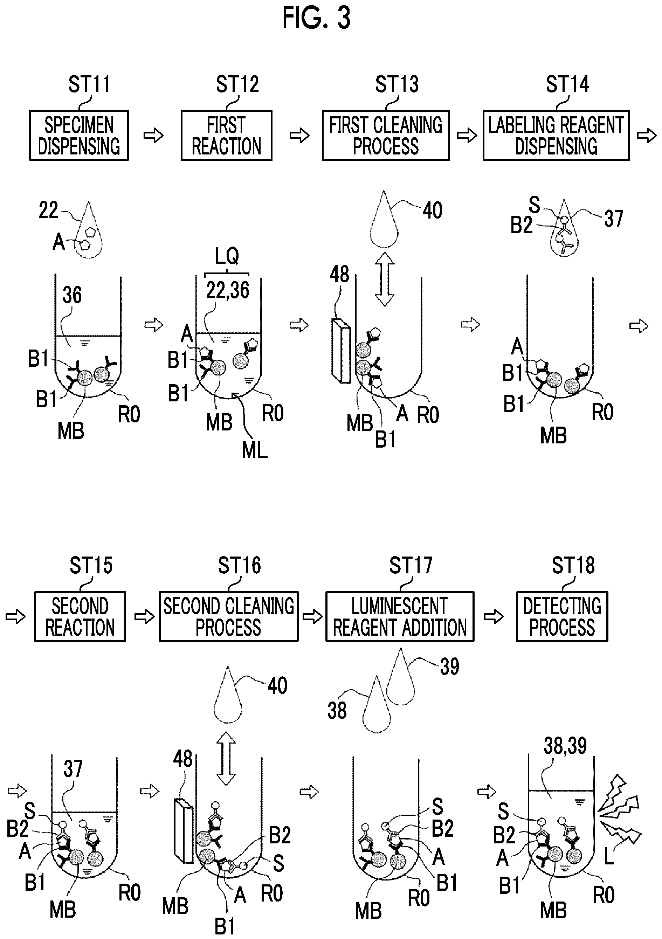

The reaction cell R0 contains a plurality of magnetic particles MB modified with a first binding substance B1 for specifically binding to the examination target substance A (see Fig. 3). The specimen 22 is dispensed into the reaction cell R0, and mixed with various reagents in the reaction cell R0. For example, in a case where the magnetic particles MB have a spherical shape, a diameter thereof is 0.1 to 10 µm, preferably 0.1 to 5 µm, and more preferably approximately 1 to 3 µm.

The cell R1 contains a buffer solution 36. The cell R2 contains a labeling reagent 37 containing a label S modified with a second binding substance B2 for specifically binding to the examination target substance A. A first luminescent reagent 38 is contained in the cell R3, and a second luminescent reagent 39 is contained in the cell R4. In the present example, the label S is an enzyme, and the label S emits light in presence of the first luminescent reagent 38 and the second luminescent reagent 39. The buffer solution 36 in the cell R1, the labeling reagent 37 in the cell R2, the first luminescent reagent 38 in the cell R3, and the second luminescent reagent 39 in the cell R4 are simply referred to as reagents 36 to 39, in a case where it is not necessary to distinguish the respective liquids.

The first binding substance B1 and the second binding substance B2, which specifically bind to the examination target substance A, are, for example, an antibody against an antigen in a case where the examination target substance A is the antigen, an antigen against an antibody in a case where the examination target substance A is the antibody, and an aptamer against a protein, a low-molecular-weight compound, or the like in a case where the examination target substance A is the protein, the low-molecular-weight compound, or the like. The first binding substance B1 and the second binding substance B2 may be the same or different from each other.

Here, an example of an examination procedure based on the chemiluminescent enzyme immunoassay method will be described with reference to Fig. 3. Fig. 3 schematically shows a reaction in a case where the specimen 22 contains the examination target substance A.

The reaction cell R0 contains the magnetic particles MB modified with the first binding substance B1 in advance, and a buffer solution 36 is dispensed into the reaction cell R0 before the dispensing of the specimen 22. In this state, the specimen 22 is dispensed into the reaction cell R0 (step ST11).

In the reaction cell R0, the magnetic particles MB, the specimen 22, and the buffer solution 36 are mixed with each other, and as a first reaction, a binding reaction in which the examination target substance A in the specimen 22 and the first binding substance B1 are specifically bound to each other occurs (step ST12). In the first reaction, the examination target substance A in the specimen 22 binds to the first binding substance B1, so that the examination target substance A is captured by the magnetic particles MB through the first binding substance B1. The mixing process of the specimen 22 and the buffer solution 36, which are the magnetic particles MB and the liquid LQ, in the step ST12 is performed by repeating suction and discharge of these mixed liquids ML by a nozzle 42 described later.

Next, a first cleaning process (B/F separation) for removing unreacted components other than the examination target substance A, which are captured by the magnetic particles MB, is performed (step ST13). A magnet 48 is disposed close to an outside of the reaction cell R0, a reaction liquid after the first reaction is discharged in a state in which the magnetic particles MB are collected on an inner wall surface of the reaction cell R0, and a cleaning liquid 40 is dispensed into the reaction cell R0. In the step ST13 in Fig. 3, a bidirectional arrow in the up-down direction shown at the upper portion of the reaction cell R0 schematically indicates a state in which the cleaning liquid 40 is dispensed into the reaction cell R0 and discharged from the reaction cell R0. In the first cleaning process, the dispensing and the discharging of the cleaning liquid 40 are repeated a plurality of times. The cleaning liquid 40 is also discharged from the reaction cell R0 in a state in which the magnet 48 is disposed close to the outside of the reaction cell R0 and the magnetic particles MB are collected on the inner wall surface of the reaction cell R0. In a case where the cleaning liquid 40 is dispensed, the mixing process is performed such that the cleaning liquid 40 and the magnetic particles MB are sufficiently mixed.

After the first cleaning process, the labeling reagent 37 is dispensed into the reaction cell R0 (step ST14). The labeling reagent 37 contains a second binding substance B2 to which the label S is imparted, which is a binding substance for specifically binding to the examination target substance A.

In the reaction cell R0, the magnetic particles MB and the labeling reagent 37 are mixed with each other, and as a second reaction, a binding reaction in which the examination target substance A captured by the magnetic particles MB and the second binding substance B2 are specifically bound to each other (step ST15). As a result, the label S is imparted to the examination target substance A through the second binding substance B2. Even in the step ST15, the mixing process is performed such that the magnetic particles MB and the labeling reagent 37 are sufficiently mixed.

Next, a second cleaning process (B/F separation) for removing unreacted components other than the second binding substance B2 bound to the examination target substance A, which are captured by the magnetic particles MB in the labeling reagent 37, is performed (step ST16). The second cleaning process (step ST16) is performed in the same manner as the first cleaning process (step ST13).

Thereafter, the first luminescent reagent 38 and the second luminescent reagent 39 are added to the reaction cell R0 (step ST17). The label S is an enzyme, and causes a chemiluminescence L in the presence of the first luminescent reagent 38 and the second luminescent reagent 39, containing a luminescent substrate. The examination target substance Ais detected by detecting the chemiluminescence L (step ST18). The examination procedure is as described above.

As an example, the examination apparatus 10 includes a specimen transporting unit 12, a mixing unit 13, a dispensing mechanism 14, a detecting unit 15, a processor 16, a memory 17, and a touch panel display 18.

In the detecting unit 15, a detection process of detecting the examination target substance A in the specimen 22 is executed. The detecting unit 15 includes a photodetector 50 such as a photomultiplier tube and a photodiode. The photodetector 50 is disposed to face the reaction cell R0, and detects the chemiluminescence L caused by the label S bound to the examination target substance A. In the present example, an enzyme is used as the label S to detect the chemiluminescence L generated by reacting with the luminescent substrate.

The processor 16 integrally controls each unit of the examination apparatus 10. An example of the processor 16 is a central processing unit (CPU) which performs various types of control by executing a program. The CPU functions as a control unit which controls each unit by executing a program.

In addition, the processor 16 acquires information on a light amount of the chemiluminescence L detected by the photodetector 50, and calculates a density of the examination target substance A based on the information on the light amount.

The memory 17 is an example of a memory connected to or built into the CPU as the processor 16. For example, the memory 17 stores a control program. In addition to the control program, the memory 17 stores setting information that is preset in order for the processor 16 to perform the various types of control.

In addition, the memory 17 stores information indicating a correspondence relationship between the light amount of the chemiluminescence L detected by the photodetector 50 and the amount (density) of the examination target substance A. The correspondence relationship is stored, for example, as a calibration curve represented by a function. The correspondence relationship may be a format of a table. The processor 16 calculates the amount of the examination target substance A from, for example, the light amount of the chemiluminescence L acquired from the photodetector 50, and the calibration curve stored in the memory 17.

The touch panel display 18 receives, from a user, an operation instruction such as an instruction to start the examination. In addition, the touch panel display 18 displays information such as an examination result.

The specimen transporting unit 12 has a loading section (not shown) into which the specimen collection container 20 for accommodating the specimen 22 is loaded, and transports the loaded specimen collection container 20 to a position accessible by a nozzle 42 of the dispensing mechanism 14, which will be described later.

The mixing unit 13 includes a loading section (not shown) into which the cartridge RC is loaded, the cartridge RC, a reagent dispensing section 13A. The mixing unit 13 performs the mixing process of mixing the particles and the liquid in the reaction cell R0, which is performed before the detecting process is performed and is performed on the specimen 22 to be subjected to the detecting process. Specifically, the step ST11 to the step ST17 in the examination procedure described with reference to Fig. 3 is performed in the mixing unit 13. The reagent dispensing section 13A includes a suction-discharge mechanism and a moving mechanism of a nozzle for reagent dispensing. Configurations of the suction-discharge mechanism and the moving mechanism are substantially the same as a suction-discharge mechanism 41 and a moving mechanism 46 in the dispensing mechanism 14, which will be described later. The reagents 36 to 39 are each suctioned from each of the cells R1 to R4 of the cartridge RC by the nozzle for reagent dispensing, and dispensed into the reaction cell R0.

The mixing unit 13 further includes a transporting section 13B of the cartridge RC. The transporting section 13B transports the cartridge RC to a location at which each process step in the mixing unit 13 is executed. Furthermore, the transporting section 13B transports the cartridge RC from the mixing unit 13 to the detecting unit 15.

The dispensing mechanism 14 includes a suction-discharge mechanism 41 and a moving mechanism 46. The suction-discharge mechanism 41 includes a nozzle 42, and is a mechanism which causes the nozzle 42 to perform a suction operation of suctioning the liquid and a discharge operation of discharging the liquid from the nozzle 42. The suction-discharge mechanism 41 includes a pump or the like, which generates a driving force for the suction and discharge. The nozzle 42 is a nozzle to be inserted into the reaction cell R0, and the suction-discharge mechanism 41 executes the mixing process of mixing the particles and the liquid by repeating the suction and discharge by the nozzle 42 in the reaction cell R0. Specifically, in the step ST12 of Fig. 3, the mixing process of suctioning and discharging the mixed liquid ML of the magnetic particles MB, the buffer solution 36, and the specimen 22 is executed. In addition, the suction-discharge mechanism 41 also functions as a specimen dispensing mechanism which dispenses the specimen by suctioning the specimen 22 from the specimen collection container 20 and discharging the specimen into the cartridge RC loaded in the mixing unit 13. The moving mechanism 46 is a mechanism which three-dimensionally moves the nozzle 42 in a vertical direction (up-down direction). The moving mechanism 46 includes an actuator such as a motor, which generates a driving force, and a driving force transmission mechanism consisting of a gear, a drive belt, and the like.

In addition, as an example, the nozzle 42 includes a nozzle body and a transparent chip 42A which is interchangeably attached to a distal end of the nozzle body. The chip 42A is replaced in order to prevent contamination from being caused by a plurality of liquids. The chip 42A is a single-use type and is disposable. The chip 42A of the nozzle 42 is replaced for each specimen 22.

The examination apparatus 10 includes a nozzle imaging camera 62 which optically images the nozzle 42, and a reaction cell imaging camera 64 which optically images the reaction cell R0.

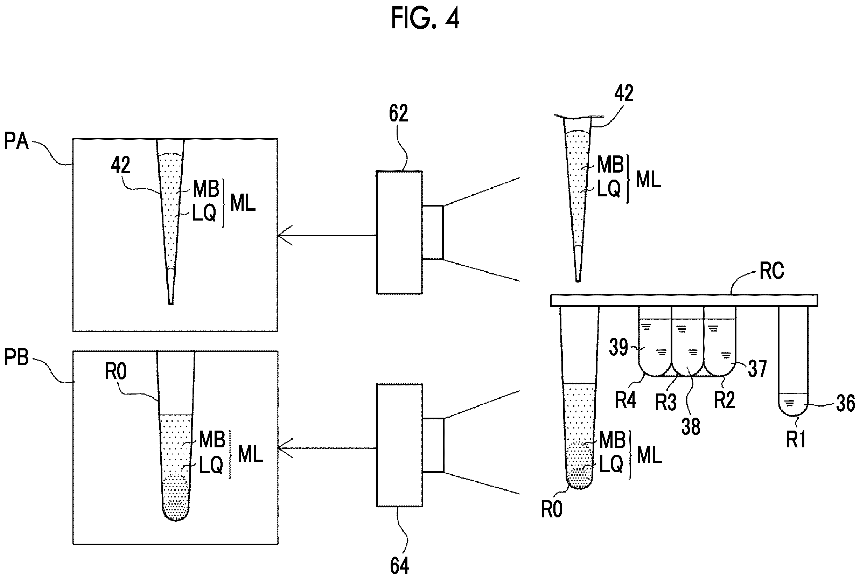

As shown in Fig. 4, the nozzle imaging camera 62 is disposed to be able to image the nozzle 42 containing the mixed liquid ML in which the magnetic particles MB and the liquid LQ are mixed in the reaction cell R0, the mixed liquid ML being suctioned from the reaction cell R0. The nozzle imaging camera 62 images the nozzle 42 containing the mixed liquid ML, and outputs a captured image PA. The nozzle imaging camera 62 is an optical camera, and includes an image sensor which images the nozzle 42 and an optical system which forms an image of the nozzle 42 on an imaging plane of the image sensor. The image sensor is a complementary metal oxide semiconductor (CMOS) image sensor, a charge coupled device (CCD) image sensor, or the like.

In addition, as shown in Fig. 4, the reaction cell imaging camera 64 is disposed at a position where the mixed liquid ML in the reaction cell R0 can be imaged. The reaction cell imaging camera 64 images the mixed liquid ML in the reaction cell R0, and outputs a captured image PB. The reaction cell imaging camera 64 is an optical camera, and includes an image sensor which images the mixed liquid ML in the reaction cell R0 and an optical system which forms an image of the mixed liquid ML on an imaging plane of the image sensor. The image sensor is a complementary metal oxide semiconductor (CMOS) image sensor, a charge coupled device (CCD) image sensor, or the like.

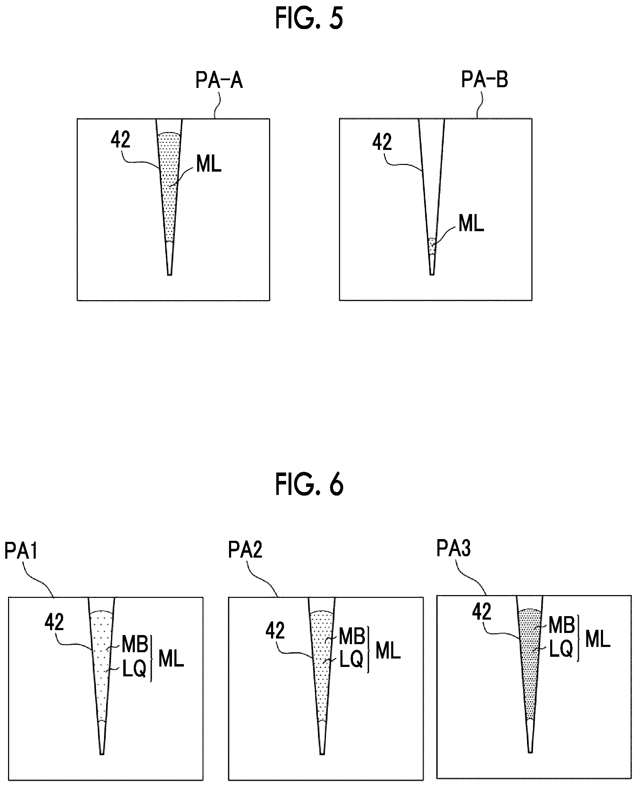

The processor 16 acquires the image PA from the nozzle imaging camera 62. The image PA includes information on a color of the mixed liquid ML in the nozzle 42, which contains the particles (here, the magnetic particles MB) and the liquid (here, the specimen 22 and the buffer solution 36) mixed in the reaction cell R0 and is suctioned from the reaction cell R0 to the nozzle 42. The processor 16 determines a mixing state of the mixed liquid ML in the reaction cell R0 from the information on the color of the mixed liquid ML in the image PA. As shown in Fig. 5, the processor 16 may acquire, as the image PA of the nozzle 42 containing the mixed liquid ML, an image PA-A of the nozzle in a state of suctioning the mixed liquid ML or an image PA-B of the nozzle containing the mixed liquid ML remaining after discharging the mixed liquid ML. It is preferable that the processor 16 acquires the image of the nozzle 42 which contains the mixed liquid ML suctioned by the last suction in the mixing process by suctioning and discharging the mixed liquid ML by the nozzle 42, or acquires the image of the nozzle 42 which contains the mixed liquid ML remaining after the last discharge performed after that.

The magnetic particles MB have a size of a submicron to a micron order, so that it is difficult to discriminate the individual magnetic particles MB in the mixed liquid ML. An amount of the magnetic particles MB provided in the reaction cell R0 is known in advance, and amounts of the buffer solution 36 and the specimen 22 dispensed are also substantially constant. In a case where the magnetic particles MB are sufficiently dispersed in the mixed liquid ML, that is, in a case where the magnetic particles MB are sufficiently mixed, the color of the mixed liquid ML is within a regulation range. On the other hand, in a case where the dispersion of the magnetic particles MB is not sufficient, too light color and too dark color, out of the regulation range, are exhibited in most of the mixed liquid ML. Therefore, in a case where the color of the mixed liquid ML in the nozzle 42 suctioned from the reaction cell R0 is within a regulation range, the processor 16 determines that the mixing state of the mixed liquid ML in the reaction cell R0 is favorable. In addition, in a case where the color of the mixed liquid ML in the nozzle 42 suctioned from the reaction cell R0 is out of a regulation range, the processor 16 determines that the mixing state of the mixed liquid ML in the reaction cell R0 is defective. In the present specification, "within a range" includes a boundary which defines the range, and "out of a range" is a region beyond the boundary.

Fig. 6 schematically shows images PA1 to PA3 of the nozzle 42 containing the mixed liquids ML of different colors. The mixed liquid ML in the image PA1 has a light color, and the color of the mixed liquid ML is darker in the image PA2 and the image PA3. For example, the color of the mixed liquid ML in the image PA2 is a color within the regulation range, and the colors of the mixed liquids ML in the image PA1 and the image PA3 are colors out of the regulation range. In this way, the mixing state of the mixed liquid ML in the reaction cell R0 is determined based on the color of the mixed liquid ML contained in the nozzle 42.

More specific examples of the information on the color of the mixed liquid ML include a pixel value of pixels representing the mixed liquid ML in the image PA. In a case of determining the goodness or badness of the mixing state using the pixel value, the processor 16 first derives an average value of pixel values of pixels of all or a part of a region representing the mixed liquid ML.

Basically, the liquid amount and the magnetic particle amount including the specimen 22 and the buffer solution 36 are examined in a certain range centered on a specified value, for example, in a range of the specified value ± 5% or the specified value ± 3%. Therefore, the concentration of the magnetic particles MB in the mixed liquid ML is also within a certain range centered on a specified value. Therefore, the average value of the pixel values of the pixels representing the mixed liquid ML in a favorable mixing state in which the magnetic particles MB are uniformly dispersed is within a certain range of pixel value. The range of pixel value taken by the average value of the pixel values of the pixels representing the mixed liquid ML in a state in which the mixing state is favorable is stored in the memory 17 as the regulation range. For example, since the magnetic particles MB are black, the image PA is a grayscale image. In this case, the pixel value is represented by 256 gradations of from 0 (black) to 255 (white). The regulation range of the average value of the pixel values representing the mixed liquid ML in a case where the magnetic particles MB are uniformly dispersed is, for example, 220 to 240. The processor 16 determines the mixing state of the mixed liquid ML in the reaction cell R0, based on the above-described average value derived from the image PA and the regulation range stored in the memory 17. Specifically, in a case where the above-described average value is within the regulation range, the processor 16 determines that the mixing state of the mixed liquid ML in the reaction cell R0 is favorable, and in a case where the above-described average value is out of the regulation range, the processor 16 determines that the mixing state of the mixed liquid ML in the reaction cell R0 is defective.

In addition, as the method of determining the goodness or badness of the mixed liquid ML from the image PA of the nozzle 42 containing the mixed liquid ML, the following method may be used. The nozzle 42 is imaged before the nozzle 42 suctions the specimen 22 and discharges the specimen 22 into the reaction cell R0. Thereafter, the mixing process is performed by the suction and discharge, and the nozzle 42 in a state of containing the mixed liquid ML suctioned in the last suction is imaged. The processor 16 acquires an image of the nozzle 42 containing the specimen 22 (this is referred to as a first image), and an image of the nozzle 42 containing the mixed liquid ML in the last suction (this is referred to as a second image). The processor 16 calculates a difference between the color of the specimen 22 in the first image and the color of the mixed liquid ML in the second image, and compares the difference in color with a preset threshold value. In a case where the difference is within a preset regulation range, such as a threshold value ± 5% or a threshold value ± 3%, it is determined that the mixing state is favorable, and in a case where the difference is out of the regulation range, it is determined that the mixing state is defective. The preset threshold value is, for example, a value determined by acquiring the first image and the second image and calculating the difference in color therebetween in calibration of the calibration curve which is obtained by performing the above-described examination procedure using a sample for calibration curve, and it is sufficient that the preset threshold value is stored in the memory 17 in the calibration.

In a case where the mixing state of the mixed liquid ML in the nozzle 42 suctioned from the reaction cell R0 is defective, the processor 16 acquires information on a color of the mixed liquid ML in the reaction cell R0. The processor 16 re-determines a mixing state of the mixed liquid ML based on the information on the color of the mixed liquid ML in the reaction cell R0. Specifically, in a case where it is determined that the mixing state is defective, the processor 16 controls the reaction cell imaging camera 64 to image the mixed liquid ML in the reaction cell R0.

The image PA of Fig. 4 shows an example in which the color of the mixed liquid ML is lighter than the regulation range, and the processor 16 determines that the mixing state is defective. For example, as shown in Fig. 4, in a case where the mixed liquid ML in the reaction cell R0 is dark on a bottom part side and has a light color range on an upper part, the mixed liquid ML in the light color region is suctioned. As shown in Fig. 4, the processor 16 acquires, from the reaction cell imaging camera 64, the image PB of the mixed liquid ML in the reaction cell R0, which includes the information on the color of the mixed liquid ML in the reaction cell R0. The processor 16 re-determines the mixing state based on the shading in the image PB of the mixed liquid in the reaction cell R0, the shading being generated due to dispersion of the particles in the mixed liquid ML.

In the reaction cell R0, in a case where the magnetic particles MB are uniformly dispersed in the mixed liquid ML, the mixed liquid ML has a uniform density as a whole, and in a case where the dispersion is non-uniform, the mixed liquid ML has shading as shown in the image PB of Fig. 4. In the image of the mixed liquid ML, a portion where the density of the magnetic particles MB is low is light in color, and a portion where the density of the magnetic particles MB is high is dark in color. The processor 16 determines the mixing state of the mixed liquid ML based on the shading generated in the image of the mixed liquid ML due to the dispersion of the magnetic particles MB in the mixed liquid ML.

The image analysis method for the processor 16 to determine the goodness or badness of the mixing state based on the shading of the image PB is not particularly limited. For example, each pixel value may be histogrammed, the number of pixels having a pixel value more than a preset threshold value may be counted, and the goodness or badness may be determined by the number of counted pixels. In addition, for example, dispersion (standard deviation) of the pixel values may be calculated, and the goodness or badness may be determined based on whether or not the standard deviation is equal to or less than a preset threshold value. The memory 17 stores information necessary for determining whether or not the mixing state of the mixed liquid ML is favorable or defective based on the image PB of the mixed liquid ML in the reaction cell R0, as setting information.

The determination of whether or not the mixing state is favorable may be performed using a machine learning model which has been subjected to a learning process using supervised training data in which the determination of whether or not the mixing state is favorable has been made.

In a case where the mixing state is determined to be favorable, the processor 16 executes detection of the examination target substance A performed by the detecting unit 15, after the process in the mixing unit 13. In contrast, in a case where the mixing state is determined to be defective, the processor 16 displays that the mixing state is defective on the touch panel display 18, and terminates the examination.

In a case where the processor 16 determines that the mixing state of the mixed liquid ML is defective, the touch panel display 18 displays an error message indicating that the mixing state is defective. That is, the touch panel display 18 functions as a notification unit which issues a notification of an error. In addition, as the notification unit which issues the notification of the error, a speaker which issues the notification of the error by voice, a light emitting unit which issues the notification of the error by emitting light, or the like may be provided separately from the touch panel display 18. In addition, instead of displaying the error message, the processor 16 may display the image PA or the image PB on the touch panel display 18, or may display the image PA or the image PB together with the error message. In a case where the image PA or the image PB is presented to the user, the processor 16 may request the user to make a determination of whether or not to continue or interrupt the examination.

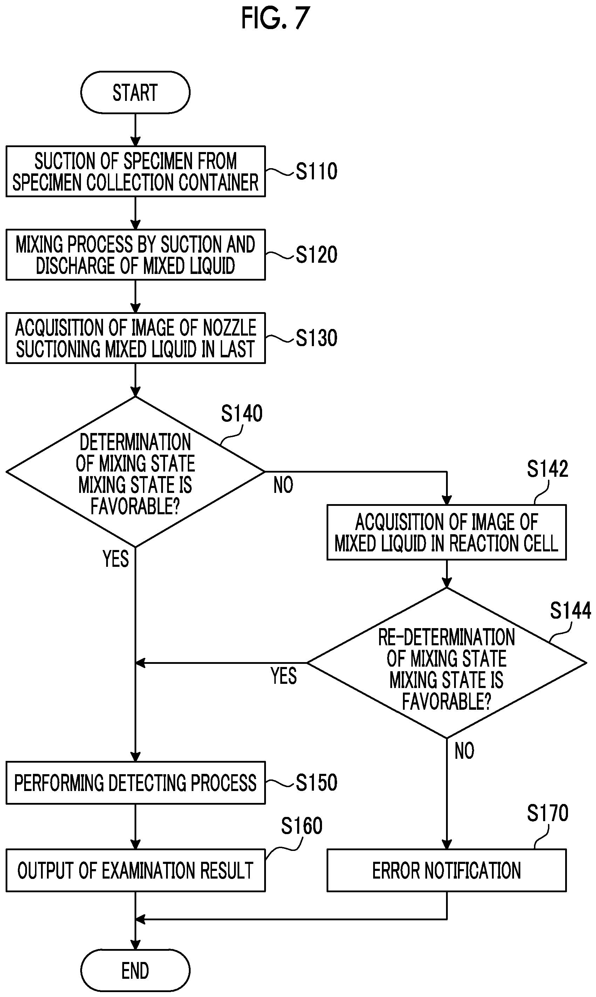

Here, an example of an examination flow in the examination apparatus 10 will be described with reference to Fig. 7 as a first examination flow. In a case where the examination is performed, the examination apparatus 10 is loaded with the specimen collection container 20 accommodating the specimen 22 to be examined and the cartridge RC.

The processor 16 controls the dispensing mechanism 14 to execute a specimen dispensing process. First, the nozzle 42 is inserted into the specimen collection container 20 to suction the specimen 22 (step S110). In a state in which the nozzle 42 contains the specimen 22, the processor 16 moves, by the moving mechanism 46, the nozzle 42 from a specimen suction position to a specimen discharge position above the reaction cell R0 to discharge the specimen 22 into the reaction cell R0. Then, the processor 16 controls the suction-discharge mechanism 41 to perform the mixing process in which the suction and discharge of the mixed liquid ML by the nozzle 42 is repeated (step S120). The suction and discharge are executed a preset number of times (hereinafter, referred to as an initial setting number of times). The initial setting number of times of the suction and discharge is stored in the memory 17.

The processor 16 controls the nozzle imaging camera 62 to image the nozzle 42 containing the mixed liquid ML suctioned by the last suction with the nozzle 42, and acquires the image PA imaged by the nozzle imaging camera 62 (step S130). Here, the image imaged by the nozzle imaging camera 62 may be an image of the nozzle 42 containing the mixed liquid ML remaining after the last discharge.

The processor 16 determines the mixing state of the mixed liquid ML in the reaction cell R0, based on the image PA (step S140). In a case where the processor 16 determines that the mixing state of the mixed liquid ML is favorable (step S140: YES), the processor 16 continues the step according to the above-described examination procedure and executes the detecting process (step S150). The processor 16 calculates the density of the examination target substance A in the specimen 22 based on the information on the light amount acquired from the photodetector 50, and outputs the examination result (step S160). Specifically, the examination result is displayed on the touch panel display 18.

On the other hand, in a case where it is determined that the state of the mixed liquid ML is defective (step S140: NO), the processor 16 controls the reaction cell imaging camera 64 to image the mixed liquid ML in the reaction cell R0, and acquires the image PB imaged by the reaction cell imaging camera 64 (step S142). The processor 16 re-determines the mixing state of the mixed liquid ML in the reaction cell R0, based on the image PB of the mixed liquid ML in the reaction cell R0 (step S144). As described above, the processor 16 re-determines the mixing state of the mixed liquid ML based on, for example, the shading of the mixed liquid ML in the reaction cell R0.

In the re-determination of the mixed liquid ML in the reaction cell R0, in a case where the processor 16 determines that the mixing state is favorable (step S144: YES), the processor 16 continues the step according to the above-described examination procedure and executes the detecting process (step S150). Thereafter, the processor 16 calculates the density of the examination target substance A in the specimen 22 based on the information on the light amount acquired from the photodetector 50, and outputs the examination result (step S160).

In the re-determination of the mixed liquid ML in the reaction cell R0, in a case where the processor 16 determines that the mixing state is defective (step S144: NO), the processor 16 outputs an error message indicating that the mixing state is defective on the touch panel display 18 (step S170), and terminates the examination.

In the examination apparatus 10 described above, as the examination apparatus including the mixing process of the particles and the liquid, in a case where the mixing of the particles and the liquid is insufficient, the density of the examination target substance A detected by the detecting process may deviate from the actual density. As a result, reliability of the examination result is reduced.

In the examination apparatus 10 according to the present embodiment, the processor 16 acquires the information on the color of the mixed liquid ML in the nozzle 42, which contains the particles (here, the magnetic particles MB) and the liquid (here, the specimen 22 and the buffer solution 36) mixed in the reaction cell R0 and is suctioned from the reaction cell R0 to the nozzle 42. The processor 16 determines the mixing state of the mixed liquid ML in the reaction cell R0 from the information on the color. With the present configuration, the mixing state of the mixed liquid ML in the reaction cell R0 can be grasped. In a case where the mixing state is defective, a countermeasure such as a notification of an error, prompting of re-measurement, and re-execution of the mixing process can be performed, and an examination result with high reliability can be obtained.

In the above-described embodiment, the mixing state in the mixing process is determined in the first reaction (step ST12 in Fig. 3) in which the specimen 22, the buffer solution 36, and the magnetic particles MB are mixed with each other in the reaction cell R0 by repeating the suction and discharge of the mixed liquid ML using the nozzle 42 (corresponding to a sampling nozzle) for dispensing the specimen 22. In addition, in each of the first cleaning process (step ST13 in Fig. 3), the second reaction (step ST15 in Fig. 3), the second cleaning process (step ST16 in Fig. 3), and the luminescent reagent addition (step ST17 of Fig. 3) in the examination procedure, the mixing process of mixing the reagents 37 to 39 or the cleaning liquid 40 with the magnetic particles MB is performed. In these steps, in a case where the mixing process is performed by suction and discharge using a nozzle having a transparent distal end and capable of observing the mixed liquid ML in the nozzle, the image of the nozzle 42 may be acquired in each step, and the mixing state of the mixed liquid ML in each step may be determined.

In the present embodiment, the processor 16 derives the density of the color from the information on the color of the mixed liquid ML (in the present example, the image PA imaged by the nozzle imaging camera 62), and in a case where the density of the color is out of a preset regulation range, the processor 16 determines that the mixing state is defective. With such a determination method, the processor 16 in the determination process has a small processing load.

In the present embodiment, the nozzle imaging camera 62 for optically imaging the nozzle 42 is provided, in which the processor 16 acquires the image PA of the nozzle 42 containing the mixed liquid imaged by the nozzle imaging camera 62, the image including the information on the color of the mixed liquid. The information on the color of the mixed liquid is not limited to the image in which the mixed liquid is contained. Instead of the nozzle imaging camera 62, an absorbance meter may be provided, and an absorbance of the mixed liquid ML may be measured by detecting transmitted light by allowing light having a specific wavelength to be incident on the mixed liquid ML contained in the nozzle 42. In this case, the absorbance corresponds to the information on the color of the mixed liquid. As a method of acquiring the information on the color of the mixed liquid, in a case of including the absorbance meter, a wavelength of light to be incident may be set according to the type of the particles. For example, in a case where the particles are the magnetic particles MB, the wavelength of light to be incident is 685 nm. In the determination of the mixing state by the absorbance, in a case where the magnetic particles MB are uniformly dispersed, that is, in a case where the mixing state is favorable, the range of the absorbance is stored in the memory 17 as a preset regulation range. It is sufficient that the processor 16 determines the goodness or badness of the mixing state depending on the measured absorbance within the regulation range.

In the present embodiment, the magnetic particles MB are used as the particles, but the particles used in the examination by the antigen-antibody reaction may be latex particles, gold colloidal particles, or the like. The technology of the present disclosure can also be applied to these particles. The magnetic particles MB are black, but the latex particles are white and the gold colloidal particles change from yellow to red to blue depending on the particle diameter. Therefore, in a case where the latex particles or the gold colloidal particles are used, it is preferable that the image to be acquired is a color image. In addition, in a case of the absorbance measurement, light having a wavelength corresponding to each color may be incident.

In a case where the mixing state of the mixed liquid ML in the reaction cell R0 is determined to be defective, the processor 16 may be configured to acquire information on a color of the mixed liquid ML in the reaction cell R0, and configured to re-determine the mixing state of the mixed liquid based on the information on the color of the mixed liquid in the reaction cell R0.

In a case where the mixing state is determined to be defective from the mixed liquid contained in the nozzle 42, the mixing state of the mixed liquid ML is re-determined based on the information on the color of the mixed liquid ML of the particles and the liquid in the reaction cell. As a result, the mixing state of the mixed liquid ML in the reaction cell R0 can be more accurately determined, and an appropriate countermeasure can be taken according to the mixing state.

In addition, in the present embodiment, the reaction cell imaging camera 64 for optically imaging the reaction cell R0 is provided. In a case where the mixing state is determined to be defective, the processor 16 controls the reaction cell imaging camera 64 to image the mixed liquid in the reaction cell R0, and acquires the image PB of the mixed liquid in the reaction cell R0. The processor 16 re-determines the mixing state based on the shading in the image of the mixed liquid ML in the reaction cell R0, the shading being generated due to dispersion of the particles in the mixed liquid ML. That is, in a case where the mixing state of the mixed liquid is determined to be defective from the mixed liquid in the nozzle 42, the mixing state of the mixed liquid is re-determined from the shading (color unevenness) of the mixed liquid ML in the reaction cell R0. As a result, the mixing state of the mixed liquid ML in the reaction cell R0 can be more accurately determined, and an appropriate countermeasure can be taken according to the mixing state.

In the configuration in which the processor 16 determines the mixing state based on the shading generated in the image of the mixed liquid ML due to the dispersion of the particles in the mixed liquid ML, the mixing state can be easily determined.

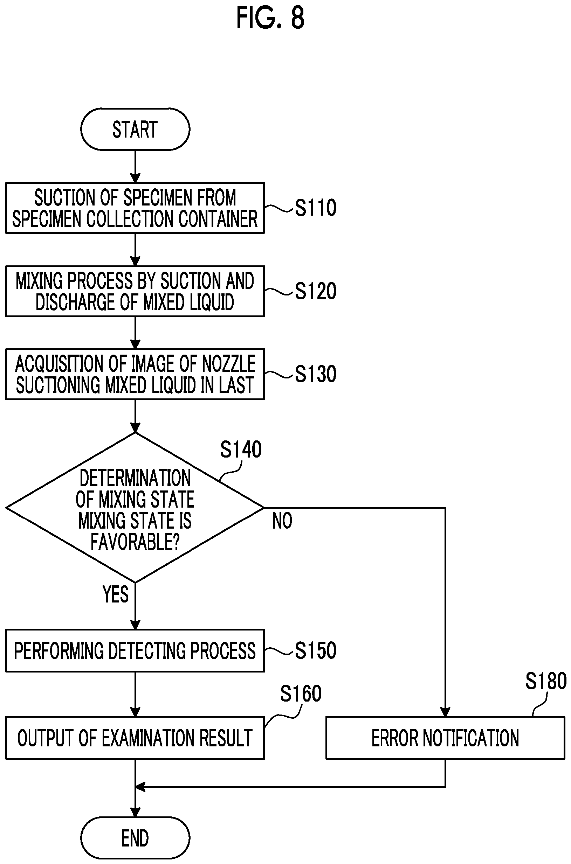

In this manner, in the above-described embodiment, the configuration in which the reaction cell imaging camera 64 is provided, and the processor 16 controls the reaction cell imaging camera 64 to image the mixed liquid in the reaction cell R0 in a case where the mixing state is determined to be defective has been described. However, the reaction cell imaging camera 64 may not be provided. Fig. 8 shows an example of an examination flow in a case where the reaction cell imaging camera 64 is not provided, as a second examination flow. In Fig. 8, the same steps as those shown in Fig. 7 are designated by the same reference numerals. As shown in Fig. 8, in the second examination flow in which the reaction cell imaging camera 64 is not provided, in a case where the mixing state is determined to be defective, an error is notified (step S170), and the examination is terminated without performing the step S142 and the step S144.

However, it is preferable that the reaction cell imaging camera 64 is provided and the step S144 of re-determining the mixing state is included, because the mixing state can be more accurately grasped.

As described above, the present examination apparatus 10 includes a notification unit (for example, the touch panel display 18) which issues a notification of an error. In a case where the mixing state is determined to be defective, the processor 16 causes the notification unit to issue the notification of the error, and terminates the examination. With such a configuration, it is possible to make the user recognize that an error has occurred due to the failure in mixing, and to clarify a countermeasure such as a re-examination.

In a case where the mixing state is determined to be defective, the processor 16 may perform a process of repeating the suction and discharge again, instead of notifying the error and terminating the examination. For example, in a case where the processor 16 determines that the mixing state of the mixed liquid in the reaction cell R0 is defective, the processor 16 restarts the suction and discharge of the mixed liquid by the nozzle 42 to promote the mixing of the particles and the liquid. The mixed liquid may be imaged again by the nozzle imaging camera 62, the processor 16 may determine the mixing state, and the mixing and the determination of the mixing state may be repeated until the mixing state of the mixed liquid to be favorable. In this case, in a case where the mixing state is still defective even though the suction and discharge and the imaging of the mixed liquid are repeated a certain number of times, an error may be notified and the examination may be terminated.

In addition, in the embodiment, the processor 16 performs the imaging of the nozzle 42 using the nozzle imaging camera 62 at the last suction or after the last discharge. The processor 16 may further cause the nozzle imaging camera 62 to capture the image of the nozzle during the mixing process by the suction and discharge of the mixed liquid for acquiring the image of the nozzle during the mixing process. In this case, the processor 16 may acquire the image of the nozzle during the mixing process, and derives a mixing state of the mixed liquid during the mixing process based on the image of the nozzle during the mixing process. A method of deriving the mixing state of the mixed liquid by the processor 16 is not limited, but for example, as in the case of the above-described embodiment, it is sufficient to derive the color of the mixed liquid and determine whether or not the mixing state is favorable or defective depending on whether or not the color of the mixed liquid is within the regulation range.

It is preferable that the processor 16 increases, by controlling the suction-discharge mechanism 41, the preset number of times of the suction and discharge in a case where the derived mixing state of the mixed liquid during the mixing process is determined not to satisfy a preset state. As a result, the mixing can be promoted to obtain a favorable mixing state. The "mixing state of the mixed liquid does not satisfy the preset state" is, as an example, a state in which the density of the color of the mixed liquid is not within the regulation range. On the other hand, in a case where the processor 16 determines that the derived mixing state of the mixed liquid during the mixing process satisfies a predetermined state, the suction and discharge may be interrupted and the processor 16 may proceed to the next step even though the number of times of suction and discharge does not reach a preset number of times.

In this way, the processor 16 can acquire the information on the color of the mixed liquid ML in the nozzle 42 during the mixing process by imaging the image of the nozzle during the mixing process by the suction and discharge of the mixed liquid, and can grasp the dispersion state (mixing state) of the particles. In a case where the processor 16 controls the suction-discharge mechanism 41 such that the number of times of suction and discharge is increased from the initial condition in a case where the mixing state during the mixing process is not sufficient, the possibility of obtaining a favorable mixing state after the mixing process is increased.

It is preferable to capture a moving image in a case of imaging the nozzle during the mixing process. The processor 16 can grasp a change in dispersion state by acquiring the moving image.

In the above-described embodiment, the nozzle imaging camera 62 and the reaction cell imaging camera 64 are provided. However, the nozzle imaging camera 62 may be able to image the reaction cell R0 at the same time as the nozzle 42. That is, the nozzle 42 and the reaction cell R0 may be imaged with one camera.

In addition, as a modification example, the processor 16 may correct the examination result of the examination target substance based on the image of the nozzle after the last discharge in the mixing process by the suction and discharge, in a case where the mixing state of the mixed liquid is determined to be favorable.

The processor 16 causes the nozzle imaging camera 62 to image the nozzle 42 after the last discharge in the mixing process by the suction and discharge of the mixed liquid, and acquires an image PA2 of the nozzle 42 after the last discharge. A small amount of the mixed liquid remains in the nozzle 42 after the last discharge, and the mixed liquid is contained in the nozzle 42. In general, a small amount of the liquid remains at the distal end of the nozzle 42 after the liquid is suctioned and discharged. The mixed liquid suctioned by the nozzle 42 contains the magnetic particles MB, the specimen 22, and the buffer solution 36. The density of the examination target substance A is derived based on a calibration curve described later, created for a predetermined amount of the specimen, but in a case where the liquid amount of the mixed liquid remaining in the nozzle 42 deviates from a calibration residual amount described later, a tolerance occurs in the detected density of the examination target substance A.

Therefore, the processor 16 derives the liquid amount of the mixed liquid remaining in the nozzle 42 after the last discharge from the image PA2 of the nozzle 42 after the last discharge. It is preferable that the processor 16 corrects the detection result of the examination target substance A based on the liquid amount of the mixed liquid remaining in the nozzle 42 and the previously acquired calibration residual amount. The calibration residual amount is a liquid amount of the liquid remaining in the nozzle 42 after the last discharge, in which the suction and discharge of the liquid (here, the mixed liquid ML) are actually performed using the nozzle 42 during the calibration.

As a premise, the nozzle 42 suctions the specimen 22 from the specimen collection container 20 by the suction-discharge mechanism 41 in a predetermined amount set in advance, and during the mixing process, the mixed liquid is suctioned and discharged, and is discharged into the reaction cell R0 to the maximum extent in the last discharge. A calibration process using a sample for calibration curve, containing a known concentration of the substance to be examined, is performed to create a calibration curve between the concentration of the substance to be examined and the light amount of the chemiluminescence L detected in the detecting process, and the calibration curve is stored in the memory 17. In the calibration process, the examination is performed according to a normal examination procedure using the sample for calibration curve as the specimen 22. In the present example, in a case where the sample for calibration curve is mixed by the suction and discharge with the magnetic particles MB and the buffer solution 36, the liquid amount of the mixed liquid remaining in the nozzle 42 after the last discharge is stored in the memory 17 as the calibration residual amount.

The liquid amount (hereinafter, referred to as a residual amount) of the mixed liquid remaining in the nozzle 42 can be derived by performing image analysis on the image PA2 obtained by imaging the nozzle 42 after the last discharge. The image analysis method is not particularly limited, but since the dimensions of the nozzle 42 are known, the liquid amount of the mixed liquid ML can be derived by detecting the lower end position and the upper end position of the mixed liquid ML in the nozzle 42.

The processor 16 corrects the examination result by comparing the residual amount derived from the image PA2 with the calibration residual amount. In a case where a difference between the residual amount and the calibration residual amount is larger than a preset threshold value, the processor 16 may issue a notification of an error and terminate the examination. Alternatively, in a case where the difference between the residual amount and the calibration residual amount is larger than a preset threshold value, the processor 16 may automatically perform the replacement of the chip 42A and the cartridge RC, and may perform the re-dispensing of the specimen 22 and the re-examination.

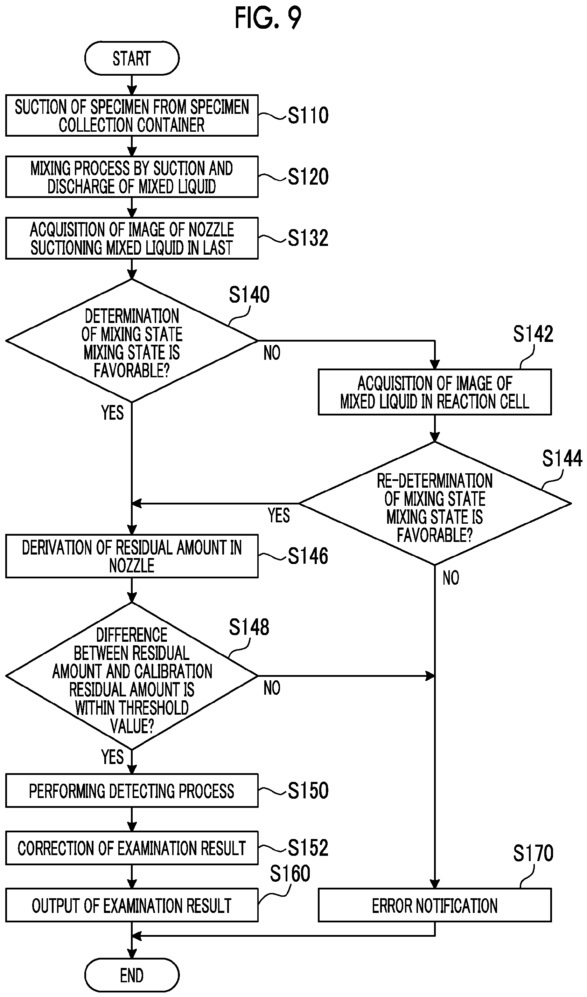

An example of an examination flow of the present modification example is shown in Fig. 9 as a third examination flow. In Fig. 9, the same steps as those of the first examination flow in Fig. 7 are denoted by the same reference numeral, and detailed description thereof will be omitted.

As shown in Fig. 9, in the third examination flow, with the processor 16, the nozzle 42 is imaged at the last discharge of the mixing process (step S120) by the suction and discharge of the mixed liquid, and the image PA-B (see Fig. 5) of the nozzle 42 containing the mixed liquid remaining in the nozzle 42 at the last discharge is acquired (step S132). The processor 16 determines the mixing state of the mixed liquid ML in the reaction cell R0 from the color of the mixed liquid ML contained by the nozzle 42 in the image PA-B (step S140). The determination method of the mixing state is as described above.

In a case where the processor 16 determines that the mixing state is defective in the determination of the mixing state based on the image PA-B (step S140: NO), the processor 16 acquires the image PB of the mixed liquid ML in the reaction cell R0 in the same manner as in the first examination flow (step S142), and re-determines the mixing state (step S144).

On the other hand, in a case where the processor 16 determines that the mixing state is favorable in the determination of the mixing state based on the image PA-B (step S140: YES), the processor 16 derives the residual amount in the nozzle 42 from the image PA-B (step S146). As described above, for example, the residual amount is derived based on the lower end position and the upper end position of the mixed liquid ML in the nozzle 42, detected from the image PA-B, and based on the dimensions of the nozzle 42 (more specifically, the chip 42A).

The processor 16 calculates a difference between the derived residual amount and the calibration residual amount, and in a case in which the difference is within a preset threshold value (step S148: YES), the processor 16 continues the examination according to the above-described examination procedure and executes the detecting process (step S150).

Thereafter, the processor 16 corrects the detection result of the examination target substance A in the detecting process based on the difference between the residual amount and the calibration residual amount (step S152). In the detecting process, the examination target substance A is optically detected. The density of the examination target substance A is obtained as the detection result based on the calibration curve between the detected light amount and the density of the examination target substance A, stored in the memory 17. Since the calibration curve is created on the premise that the specimen amount is defined, in a case where the residual amount is different from the calibration residual amount, a deviation occurs in the sample amount assumed. The density of the examination target substance A, which is the detection result, is corrected according to the deviation of the specimen amount. In the output of the examination result (step S160), the processor 16 displays the corrected detection result on the touch panel display 18.

In a case where the difference between the derived residual amount and the calibration residual amount is larger than a threshold value (step S148: NO), the processor 16 issues a notification of an error (step S170) and terminates the examination. For example, in the step S170, an error message indicating that the suction and discharge are defective due to the nozzle 42 is displayed on the touch panel display 18. In a case where the difference between the derived residual amount and the calibration residual amount is larger than the threshold value, a malfunction of the suction-discharge mechanism 41 or a malfunction of the nozzle 42 is conceivable. By notifying the error, it is possible to prompt the user to take an appropriate countermeasure such as checking the suction-discharge mechanism 41 and replacing the nozzle 42.

In this way, the processor 16 derives the liquid amount of the mixed liquid remaining in the nozzle 42 after the last discharge from the image PA-B of the nozzle 42 after the last discharge. The processor 16 corrects the detection result of the examination target substance A based on the liquid amount of the mixed liquid remaining in the nozzle 42 and the previously acquired calibration residual amount. As a result, it is possible to correct an error in the detection result caused by the difference between the residual amount and the calibration residual amount, and to obtain a more accurate detection result.

As a further modification example, in a case where the mixing state is determined to be favorable, the processor 16 may correct the detection result based on the amount of the specimen suctioned by the nozzle 42, in addition to the correction of the detection result based on the residual amount.

As an example, first, the processor 16 acquires, as a first image, an image of the nozzle 42 containing the specimen 22 before the nozzle 42 suctions the specimen 22 and discharges the specimen 22 into the reaction cell R0. Next, the processor 16 executes the mixing process by repeating the suction and discharge in the reaction cell R0 a preset number of times by the nozzle 42. The processor 16 acquires, as a second image, an image of the nozzle 42 after the last discharge of the mixed liquid ML within the preset number of times. The processor 16 determines the mixing state of the mixed liquid ML in the reaction cell R0 based on the second image, and in a case where the mixing state is determined to be favorable, the processor 16 corrects the detection result based on the first image and the second image.

Normally, it is assumed that the amount of the specimen 22 suctioned by the nozzle 42 is a predetermined amount set in advance, but the suction amount may be different from the predetermined amount in the first place due to the malfunction of the suction-discharge mechanism 41 and the like. In a case where the liquid amount of the specimen 22 contained in the nozzle 42 deviates from the predetermined amount, an error occurs in the examination result. Therefore, it is preferable that the processor 16 derives the liquid amount of the specimen 22 suctioned by the nozzle 42 from the specimen collection container 20 from the first image, calculates a difference with the predetermined amount, and corrects the examination result by multiplying the correction coefficient by the calibration curve. Together with the correction based on the first image, the residual amount of the mixed liquid in the nozzle 42 is derived from the second image which is the image of the nozzle 42 after the last discharge in the mixing process, as described in the modification example, and the detection result is further corrected based on the difference between the residual amount and the calibration residual amount.

In this way, in a case where the processor 16 determines that the mixing state is favorable, the detection result may be corrected based on the first image which is the image of the nozzle 42 containing the specimen 22 before the mixed liquid ML is discharged into the reaction cell R0 after the specimen 22 is suctioned by the nozzle 42, and the second image which is the image of the nozzle 42 after the mixed liquid ML is discharged in the last of the preset number of times. As a result, a more accurate detection result can be obtained.

In a case where the difference between the liquid amount of the specimen 22 derived from the first image and the predetermined amount is larger than a predetermined threshold value, an error may be notified to the user as a suction failure of the specimen 22 to prompt the user to take an appropriate countermeasure.

In the above-described examples, the immunological analysis apparatus which detects the examination target substance A contained in the specimen 22 by the antigen-antibody reaction has been described as an example of the examination apparatus 10. The technology of the present disclosure can be used for an examination apparatus other than the immunological analysis apparatus. In addition, the chemiluminescent enzyme immunoassay method has been described as an example of the method of detecting the examination target substance A, but the present disclosure is not limited to this method and may be applied to other methods.

In each of the above-described embodiments, as hardware structures of processing units that execute various types of process as the internal configuration of the processor 16, the following various processors can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, or a dedicated electric circuit, such as an application specific integrated circuit (ASIC), which is a processor having a dedicated circuit configuration designed to perform a specific process.

One processing unit may be constituted by one of the various processors, or may be a combination of two or more processors of the same type or different types (for example, a combination of a plurality of FPGAs and/or a combination of a CPU and an FPGA). The plurality of processing units may be configured of one processor.

As an example of the plurality of processing units composed of one processor, first, as represented by computers such as a client and a server, there is a form in which one processor is composed of a combination of one or more CPUs and software and this processor functions as a plurality of processing units. A second example of the configuration is an aspect in which a processor that implements the functions of the whole system including a plurality of processing units using one integrated circuit (IC) chip is used, and a representative example of this aspect is a system-on-chip (SoC). As described above, the various processing units are configured using one or more of the various processors as the hardware structure.

More specifically, a circuitry combining circuit elements such as semiconductor elements may be used as the hardware structure of the various processors.

The present disclosure is not limited to the above-described embodiments, and can be implemented with appropriate modifications, such as omitting a configuration or replacing a configuration with a different configuration within the scope that does not deviate from the gist of the present disclosure.

The following appendixes are further disclosed with respect to the above embodiments.

### (Appendix 1)

An examination apparatus including:
a detection unit for executing a detecting process for detecting an examination target substance in a specimen;
a suction-discharge mechanism for executing a mixing process for mixing particles and a liquid by repeating suction and discharge using a nozzle inserted in a reaction cell, the mixing process being a process which is performed before the detecting process and performed on the specimen to be subjected to the detecting process; and
a processor for acquiring information on a color of a mixed liquid in the nozzle, which contains the particles and the liquid mixed in the reaction cell and is suctioned from the reaction cell to the nozzle, and determine a mixing state of the mixed liquid in the reaction cell from the information on the color.

### (Appendix 2)

The examination apparatus according to the appendix 1,
in which the processor is configured to derive a density of the color from the information on the color of the mixed liquid and configured to determine that the mixing state is defective in a case where the density of the color is out of a preset regulation range.

### (Appendix 3)

The examination apparatus according to the appendix 1 or the appendix 2, further including:
a nozzle imaging camera configured to optically image the nozzle,
in which the processor is configured to acquire an image of the nozzle containing the mixed liquid imaged by the nozzle imaging camera, the image including the information on the color of the mixed liquid.

### (Appendix 4)

The examination apparatus according to any one of the appendixes 1 to 3,
in which, in a case where the mixing state is determined to be defective, the processor is configured to
acquire information on a color of the mixed liquid in the reaction cell, and
re-determine the mixing state of the mixed liquid based on the information on the color of the mixed liquid in the reaction cell.

### (Appendix 5)

The examination apparatus according to the appendix 4, further including:
a reaction cell imaging camera configured to optically image the reaction cell,
in which, in the case where the mixing state is determined to be defective, the processor is configured to
   image, from the reaction cell imaging camera, the mixed liquid in the reaction cell by controlling the reaction cell imaging camera, and acquire an image of the mixed liquid in the reaction cell, including the information on the color of the mixed liquid in the reaction cell, and
   re-determine the mixing state based on shading in the image of the mixed liquid in the reaction cell, the shading being generated due to dispersion of the particles in the mixed liquid.

### (Appendix 6)

The examination apparatus according to the appendix 3,
in which the processor is configured to
execute the mixing process by controlling the suction-discharge mechanism to repeat the suction and discharge in the reaction cell a preset number of times using the nozzle,
image, by controlling the nozzle imaging camera, the nozzle in a state in which the mixed liquid is suctioned in last among the preset number of times or the nozzle after the mixed liquid is discharged last, and
acquire an image of the nozzle imaged by the nozzle imaging camera.

### (Appendix 7)

The examination apparatus according to the appendix 6,
in which the processor is configured to
image the nozzle after the last discharge by controlling the nozzle imaging camera,
acquire an image of the nozzle after the last discharge,
in a case where the mixing state of the mixed liquid is determined to be favorable, derive a liquid amount of a mixed liquid remaining in the nozzle after the last discharge from the image of the nozzle after the last discharge, and
correct a detection result of the examination target substance based on the liquid amount of the mixed liquid and a previously acquired calibration residual amount of the liquid, which is an amount remaining in the nozzle after discharge for calibration by performing suction and discharge of the liquid using the nozzle.

### (Appendix 8)

The examination apparatus according to any one of the appendixes 1 to 7, further including:
a notification unit configured to issue a notification of an error,
in which, in a case where the mixing state is determined to be defective, the processor is configured to notify an error by the notification unit and terminate the examination of the specimen.

### (Appendix 9)

The examination apparatus according to the appendix 4 or the appendix 5,
in which the nozzle is a sampling nozzle which suctions the specimen and discharges the specimen into the reaction cell, and
the processor is configured to
acquire, as a first image, an image of the nozzle containing the specimen, after the specimen is suctioned by the nozzle and before the specimen is discharged into the reaction cell, and acquire, as a second image, an image of the nozzle after the mixing process is executed by repeating the suction and discharge a preset number of times in the reaction cell by the nozzle and the mixed liquid is discharged in last among the preset number of times, and
correct a detection result of the examination target substance based on the first image and the second image in a case where the mixing state of the mixed liquid is determined to be favorable.

### (Appendix 10)

The examination apparatus according to the appendix 3,
in which the processor is configured to
acquire an image of the nozzle during the mixing process, and
derive a mixing state of the mixed liquid during the mixing process based on the image of the nozzle during the mixing process.

### (Appendix 11)

The examination apparatus according to the appendix 10,
in which the processor is configured to increase, by controlling the suction-discharge mechanism, the preset number of times of the suction and discharge in a case where the derived mixing state of the mixed liquid during the mixing process is determined not to satisfy a preset state.

### (Appendix 12)

The examination apparatus according to the appendix 10 or the appendix 11,
in which the processor is configured to image a moving image of the nozzle during the mixing process by the nozzle imaging camera.

### (Appendix 13)

The examination apparatus according to the appendix 12,
in which the nozzle imaging camera is capable of simultaneously imaging the reaction cell.

### (Appendix 14)

The examination apparatus according to any one of the appendixes 1 to 13,
in which the examination target substance in the specimen is detected by an antigen-antibody reaction.

### (Appendix 15)

The examination apparatus according to the appendix 14,
in which the particles are magnetic particles modified with a binding substance specifically bound to the examination target substance.

The disclosure of Japanese Patent Application No. 2022-075197 filed on April 28, 2022 is incorporated in the present specification by reference. In addition, all documents, patent applications, and technical standards described in the present specification are incorporated herein by reference to the same extent as in a case of being specifically and individually noted that individual documents, patent applications, and technical standards are incorporated by reference.

## Claims

1. An examination apparatus comprising:
a detection unit for executing a detecting process for detecting an examination target substance in a specimen;
a suction-discharge mechanism for executing a mixing process for mixing particles and a liquid by repeating suction and discharge using a nozzle inserted in a reaction cell, the mixing process being a process which is performed before the detecting process and performed on the specimen to be subjected to the detecting process; and
a processor for acquiring information on a color of a mixed liquid in the nozzle, which contains the particles and the liquid mixed in the reaction cell and is suctioned from the reaction cell to the nozzle, and determine a mixing state of the mixed liquid in the reaction cell from the information on the color.

2. The examination apparatus according to claim 1,
wherein the processor is configured to derive a density of the color from the information on the color of the mixed liquid and configured to that the mixing state is defective in a case where the density of the color is out of a preset regulation range.

3. The examination apparatus according to claim 1, further comprising:
a nozzle imaging camera configured to optically image the nozzle,
wherein the processor is configured to acquire an image of the nozzle containing the mixed liquid imaged by the nozzle imaging camera, the image including the information on the color of the mixed liquid.

4. The examination apparatus according to claim 1,
wherein, in a case where the mixing state is determined to be defective, the processor is configured to
acquire information on a color of the mixed liquid in the reaction cell, and re-determine the mixing state of the mixed liquid based on the information on the color of the mixed liquid in the reaction cell.

5. The examination apparatus according to claim 4, further comprising:
a reaction cell imaging camera configured to optically image the reaction cell, wherein, in the case where the mixing state is determined to be defective, the processor is configured to
image, from the reaction cell imaging camera, the mixed liquid in the reaction cell by controlling the reaction cell imaging camera, and acquire an image of the mixed liquid in the reaction cell, including the information on the color of the mixed liquid in the reaction cell, and
re-determine the mixing state based on shading in the image of the mixed liquid in the reaction cell, the shading being generated due to dispersion of the particles in the mixed liquid.

6. The examination apparatus according to claim 3,
wherein the processor is configured to
execute the mixing process by controlling the suction-discharge mechanism to repeat the suction and discharge in the reaction cell a preset number of times using the nozzle,
image, by controlling the nozzle imaging camera, the nozzle in a state in which the mixed liquid is suctioned in last among the preset number of times or the nozzle after the mixed liquid is discharged last, and
acquire an image of the nozzle imaged by the nozzle imaging camera.

7. The examination apparatus according to claim 6,
wherein the processor is configured to
image the nozzle after the last discharge by controlling the nozzle imaging camera,
acquire an image of the nozzle after the last discharge,
in a case where the mixing state of the mixed liquid is determined to be favorable, derive a liquid amount of a mixed liquid remaining in the nozzle after the last discharge from the image of the nozzle after the last discharge, and
correct a detection result of the examination target substance based on the liquid amount of the mixed liquid and a previously acquired calibration residual amount of the liquid, which is an amount remaining in the nozzle after discharge for calibration by performing suction and discharge of the liquid using the nozzle.

8. The examination apparatus according to claim 1, further comprising:
a notification unit configured to issue a notification of an error, wherein, in a case where the mixing state is determined to be defective, the processor is configured to notify an error by the notification unit and terminate the examination of the specimen.

9. The examination apparatus according to claim 4,
wherein the nozzle is a sampling nozzle which suctions the specimen and discharges the specimen into the reaction cell, and
the processor is configured to
acquire, as a first image, an image of the nozzle containing the specimen, after the specimen is suctioned by the nozzle and before the specimen is discharged into the reaction cell, and acquire, as a second image, an image of the nozzle after the mixing process is executed by repeating the suction and discharge a preset number of times in the reaction cell by the nozzle and the mixed liquid is discharged in last among the preset number of times, and
correct a detection result of the examination target substance based on the first image and the second image in a case where the mixing state of the mixed liquid is determined to be favorable.

10. The examination apparatus according to claim 3,
wherein the processor is configured to
acquire an image of the nozzle during the mixing process, and
derive a mixing state of the mixed liquid during the mixing process based on the image of the nozzle during the mixing process.

11. The examination apparatus according to claim 10,
wherein the processor is configured to increase, by controlling the suction-discharge mechanism, the preset number of times of the suction and discharge in a case where the derived mixing state of the mixed liquid during the mixing process is determined not to satisfy a preset state.

12. The examination apparatus according to claim 10,
wherein the processor is configured to image a moving image of the nozzle during the mixing process by the nozzle imaging camera.

13. The examination apparatus according to claim 12,
wherein the nozzle imaging camera is capable of simultaneously imaging the reaction cell.

14. The examination apparatus according to any one of claims 1 to 13,
wherein the examination target substance in the specimen is detected by an antigen-antibody reaction.

15. The examination apparatus according to claim 14,
wherein the particles are magnetic particles modified with a binding substance specifically bound to the examination target substance.
